# EUROPÄISCHE PATENTSCHRIFT

(11) **EP 3 869 603 B1**
(45) Veröffentlichungstag und Bekanntmachung des Hinweises auf die Patenterteilung: **03.06.2026**
(21) Anmeldenummer: 21156692.2
(22) Anmeldetag: 11.02.2021
(51) Int. Cl.: H01M 10/0525, H01M 10/42, H01M 50/105, G01N 21/88, H01M 10/04, H01M 50/46, G01N 21/84, H01M 10/0585

(54) **INSPEKTIONSSYSTEM FÜR BATTERIEELEKTRODEN**
INSPECTION SYSTEM FOR BATTERY ELECTRODES
SYSTÈME D'INSPECTION POUR ÉLECTRODES DE BATTERIE

(30) Priorität: 21.02.2020 DE 102020104668
(43) Veröffentlichungstag der Anmeldung: 25.08.2021
(73) Patentinhaber: PowerCo SE, 38239 Salzgitter (DE)
(72) Erfinder: Jordan, Marco, 38229 Salzgitter (DE); Tornow, Alexander, 29369 Ummern (DE)
(74) Vertreter: Rössler, Matthias

(56) Entgegenhaltungen:
- EP-A1- 3 627 143
- EP-B1- 2 182 568
- WO-A1-2016/026782
- WO-A1-2019/190129
- DE-A1- 102013 221 592
- DE-A1- 102017 223 835
- JP-A- 2015 176 699
- US-A1- 2010 112 453

## Beschreibung

Die vorliegende Erfindung betrifft ein Verfahren zur Überprüfung eines mehrschichtigen Elektroden-Separator-Verbunds für eine Batteriezelle sowie eine Vorrichtung zur Überprüfung des Elektroden-Separator-Verbunds.

Lithium-Ionen-Batterien gelten in der modernen Technologie zunehmend als Schlüsseltechnologie. Von daher unterliegen sie einer fortlaufenden Weiterentwicklung unter unterschiedlichen Aspekten, wie beispielsweise den Herstellungskosten, der erzielbaren Energiedichte, der Lebensdauer, der Sicherheit und/oder der Ladedauer, um nur einige wenige Aspekte beispielhaft zu nennen.

Batterien zur Speicherung von größeren Energiemengen, wie beispielsweise Traktionsbatterien in Fahrzeugen, bestehen dabei in der Regel aus einer Mehrzahl von Batteriezellen, die ihrerseits aus einer Mehrzahl von Zellkomponenten zusammengesetzt sind. Zellkomponenten von Batteriezellen sind Elektroden, Separatorschichten, Stromableiterschichten bzw. Stromableiter, Schichten von Aktivmaterial an den Elektroden, Elektrolyten und umschließende Gehäuse bzw. Folien.

Die Lithium-Ionen-Batterien bzw. die nach diesem Prinzip aufgebauten Batteriezellen weisen dabei grundsätzlich zwei Elektroden auf. Zum einen ist eine negative Elektrode vorgesehen, die Anode genannt wird, sowie eine positive Elektrode, die Kathode genannt wird.

Die Elektroden bestehen dabei in der Regel aus Stromableitern und Aktivmaterial. Auf dem Elektrodensheet ist jeweils ein der Polung entsprechendes Aktivmaterial angeordnet. So wird an der Anode ein anderes Aktivmaterial verwendet als an der Kathode. Zwischen zwei Elektroden unterschiedlicher Polung ist zudem wenigstens eine Separatorschicht vorgesehen, die einen elektrischen Kurzschluss zwischen den Elektroden verhindert. Gleichzeitig aber ist die Separatorschicht für bestimmte Ionen, wie etwa Lithium-Ionen, durchlässig, so dass diese Ionen während dem Laden bzw. Entladen der Batterie jeweils durch die Separatorschicht hindurchtreten können.

Für die Funktion der Batteriezellen ist weiterhin noch ein ionenleitender Elektrolyt erforderlich, der die Aktivschichten der Elektroden und die Separatorschicht derart benetzt, dass er als Vermittler der Vorgänge in der Zelle wirkt. Die Begriffe Anode und Kathode sind über den Oxidation- bzw. Reduktionsprozess definiert. An welcher der beiden Elektroden oxidiert bzw. reduziert wird, hängt davon ab, ob die Batteriezellen geladen oder entladen werden. Es hat sich bei der Betrachtung von Batterien bzw. Batteriezellen jedoch eingebürgert immer den Entladevorgang als Definition für die Begrifflichkeiten Anode und Kathode zu verwenden.

Häufig besteht die negative Elektrode der Batteriezellen aus einer Kupferfolie und einer Schicht aus Graphit oder Lithium-legiertem Material als elektrochemisches Aktivmaterial. In ihr werden während des Ladens die für die Leistungsbereitstellung benötigten positiv geladenen Lithium-Ionen eingelagert (Interkalation). Graphitanoden sind aktuell die gängigste Wahl, da sie ein niedriges Elektrodenpotenzial und eine geringe Volumenausdehnung bei der Einlagerung von Lithium-Ionen aufweisen. Die positive Elektrode besteht häufig aus Mischoxiden, die auf einem Aluminiumkollektor aufgebracht sind. Die positive Elektrode mit dem ihr zugeordneten Aktivmaterial dient bei der Ladung der Zelle als Lithiumquelle.

Bekannte Lithium-Ionen-Batterien sind in der Regel aus einer Vielzahl von Elektrodensheets aufgebaut, die in einer Batteriezelle übereinander angeordnet sind. Bei der Herstellung von Batteriezellen ist es daher vorteilhaft zunächst mehrere der benötigten Schichten mit dem gewünschten Schichtaufbau zu Elektrodensheets miteinander zu verbinden, um anschließend in einem weiteren Fertigungsschritt aus den vorgefertigten Elektrodensheets Batteriezellen, auch Zellstapel genannt, herzustellen.

So werden beispielsweise während eines Laminationsprozesses einzelne Komponenten der Batteriezellen, wie die Anode, die Separatorschicht und die Kathode fest miteinander verbunden, sodass diese in ihrer Position und Orientierung zueinander fixiert sind. Dabei entsteht ein sogenanntes mehrlagiges Elektrodensheet, das nachfolgend als Elektroden-Separator-Verbund (ESV) bezeichnet wird. Besonders bei großformatigen Batteriezellen, wie etwa sogenannten Pouch-Zellen, stellt die Lamination ein vorteilhaftes Verfahren dar, um sowohl die Prozessgeschwindigkeit im Fertigungsprozess zu erhöhen als auch die Zellleistungsperformance im späteren Endprodukt zu verbessern. Die Erfindung beschränkt sich nicht zwingend auf einen Monozellen-Verbund (Anode, Separator, Kathode, Separator), es kann auch für einzelne laminierte Elektrodensheets (Bsp. Separator, Anode, Separator) angewendet werden.

Der Laminationsprozess stellt jedoch ein sehr komplexes Produktionsverfahren dar. Dabei müssen äußerst sensible Materialien verarbeitet werden. Besonders kritisch ist die Verarbeitung von Separatorschichten, weil diese äußerst empfindlich auf die im Prozess vorherrschenden Druck- und Temperaturbelastungen reagieren. Die Herausforderung während des Prozesses besteht darin, eine optimale Verbindung zwischen den schichtförmigen Zellkomponenten zu erreichen, ohne dabei die Zellkomponenten zu bestätigen.

Sämtliche bislang bekannte Methoden zur Verbindung der Schichten erfordern eine relativ aufwendige Prozessführung. So müssen regelmäßig stichprobenartige Kontrollen durchgeführt werden, um sicherzustellen, dass die verschiedenen Schichten zuverlässig miteinander verbunden worden sind.

Die bislang bekannten Lösungen haben somit den Nachteil, dass sie aufwendig sind und für eine Großserienfertigung nur bedingt geeignet sind. Zudem können fehlerhafte Produktionsvorgänge bis zur nächsten Probenentnahme und Auswertung relativ lange unentdeckt bleiben.

Die JP 2015-176 699 A ist auf eine Stapelvorrichtung für Elektroden und Separatoren einer Batteriezelle gerichtet.

Aufgabe der vorliegenden Erfindung ist es daher, die sich aus dem Stand der Technik ergebenden Probleme zumindest teilweise zu lösen. Insbesondere wird ein Verfahren zur Überprüfung von laminierten Elektroden-Separator-Verbunden und Batterien mit Elektroden-Separator-Verbunden anzugeben, das für eine Großserienfertigung geeignet ist und eine Überprüfung der sicheren und zuverlässigen Verbindung der Schichten miteinander gewährleistet.

Zur Lösung dieser Aufgaben wird ein Verfahren mit den Merkmalen gemäß dem unabhängigen Patentanspruch vorgeschlagen. Vorteilhafte Weiterbildungen sind Gegenstand der jeweils abhängigen Patentansprüche.

Das erfindungsgemässe Verfahren zur Überprüfung eines mehrschichtigen Elektroden-Separator-Verbunds für eine Batteriezelle, ist ein Verfahren gemäss dem unabhängigen Anspruch 1.

Das so gestaltete Verfahren ermöglicht eine lückenlose und zeitnahe Prozessüberwachung des Herstellungsprozesses.

Dazu können in einem ersten Schritt a) zunächst wenigstens zwei Funktionsschichten relativ zueinander angeordnet werden, wobei eine vorgegebene Positionierung und Orientierung der beiden Funktionsschichten eingehalten wird.

"Funktionsschichten" in diesem Sinne sind Zellkomponenten, die für die Funktion der Batteriezelle erforderlich sind, nämlich schicht- oder plattenförmige Kathoden, Anoden und Separatoren.

Unter einer "Zusammenfügen" wird hier insbesondere verstanden, wie die Funktionsschichten relativ zueinander gefügt werden. Dabei sollten die Funktionsschichten einander überdeckend berühren und richtig orientiert zueinander angeordnet sein, um in dieser Lage miteinander (dauerhaft) in Verbindung gebracht zu werden.

Die "Orientierung" der Funktionsschichten betrifft die Verwendung von Funktionsschichten, die eine bestimmte Orientierungsrichtung aufweisen. Die Orientierung kann wahlweise die Ausrichtung entlang z. B. der Breite, der Länge oder der Höhe der Funktionsschicht betreffen. So können beispielsweise Funktionsschichten verwendet werden, deren Festigkeit/Belastbarkeit in eine erste Richtung höher ist als in eine dazu querverlaufende zweite Richtung. Weiterhin können auch Funktionsschichten verwendet werden, die in Richtung der Höhe der Funktionsschicht eine Orientierung aufweisen. So können beispielsweise schichtförmige Elektroden verwendet werden, die einseitig speziell für den Kontakt mit einem entsprechenden Aktivmaterial vorbereitet sind. Diese sind dann derart orientiert anzuordnen, dass diese vorbereitete Oberfläche während des Zusammenfügens mit dem zugehörigen Aktivmaterial in Kontakt gelangt. In dem darauffolgenden bzw. nachgelagerten Schritt b) werden die so zueinander positionierten Funktionsschichten zu einem Elektroden-Separator-Verbund verbunden.

Ein "Elektroden-Separator-Verbund" wird somit als "unverlierbarer" Verbund zumindest eines Teils der in Schritt a) miteinander zusammengefügten Funktionsschichten verstanden. Der Verbund erfolgt insbesondere großflächig über die Länge und Breite der jeweiligen Funktionsschicht. Der Elektroden-Separator-Verbund kann dabei wahlweise aus Funktionsschichten mit vorgegebener Länge oder aus endlosen Bahnen von Funktionsschichten hergestellt werden, die mit dem gewünschten Schichtaufbau miteinander zusammengefügt und verbunden werden. Bei der Verwendung endloser bahnenförmiger Funktionsschichten können so zunächst endlose Elektroden-Separator-Verbunde hergestellt werden, aus denen dann durch einen Längenzuschnitt die mehrlagigen Elektrodensheets bzw. Elektroden-Separator-Verbunde mit definierter Länge gefertigt werden können. Alternativ können die Funktionsschichten auch zunächst einzeln auf die gewünschte Länge zugeschnitten und danach miteinander verbunden werden.

Nachfolgend bzw. anschließend wird in einem Schritt c) zur Qualitätssicherung die zwischen den Funktionsschichten geschaffene Verbindung kontrolliert. Dazu wird zunächst wenigstens ein Teil der Oberfläche des Elektroden-Separator-Verbunds mittels einer Erfassungsvorrichtung erfasst, um daraus ein Messergebnis zu erzeugen.

Unter einer "Oberfläche" wird hierbei eine (der beiden) äußere(n) Fläche(n) des Elektroden-Separator-Verbund verstanden, die parallel zur Fügeebene bzw. parallel zu den Funktionsschichten orientiert ist. Bevorzugt wird ein wesentlicher Teil der Oberfläche erfasst, z.B. mit einer Fläche im Bereich von 1 bis 1000 cm² bzw. von 1 bis 100.% der in Kontakt stehenden Oberfläche.

Die "Erfassungsvorrichtung" ist insbesondere geeignet, die Oberflächentopografie, Oberflächentemperatur und/oder Oberflächenfarbe zu erfassen. Dies kann mittels eines optischen Sensors, eines Fotoapparates erfolgen und erfolgt erfindungsgemäss mit einer Kamera.

Es ist möglich, dass die "Erfassungsvorrichtung" mindestens eine Beleuchtungseinheit umfasst, die auf die zu erfassende Oberfläche des Elektroden-Separator-Verbunds Licht abgeben kann.

Die Erfassungsvorrichtung kann elektronische Daten und/oder elektrische Signale als Messergebnis erzeugen und weiterleiten. Das Messergebnis kann (zwischen)gespeichert oder direkt weitergeleitet werden.

Dieses Messergebnis wird dann im nachfolgenden Schritt d) ausgewertet, um daraus wiederum ein Auswertungsergebnis zu erzeugen.

Das "Auswerten" kann ein Vergleichen, ein Verändern, ein Analysieren des Messergebnisses anhand vorgegebener Kennwerte, Funktionen und/oder Rechenroutinen umfassen. Dies kann eine Bearbeitung der elektronischen Daten und/oder elektrischen Signale umfassen. Es ist auch möglich, dass dies eine Bildbearbeitung und/oder Bildanalyse umfasst. Das Auswertungsergebnis kann (zwischen)gespeichert oder direkt weitergeleitet werden.

Dieses Auswertungsergebnis wird dann im abschließenden Schritt e) ausgegeben, um weiterverarbeitet zu werden.

Insbesondere wird das Auswertungsergebnis an eine übergeordnete Kontrolleinheit bzw. Anzeigeeinheit (außerhalb der Erfassungsvorrichtung) übergeben. Die Ausgabe erfolgt bevorzugt so, dass dieses unmittelbar bzw. automatisch in eine Anzeige und/oder eine Anpassung des Herstellungsprozesses resultieren kann.

Die Schritte c) bis e) ermöglichen es, den Herstellungsprozess fortlaufend zu überwachen und durch die unmittelbare Echtzeiterkennung von fehlerhaften Elektroden-Separator-Verbunden eine gleichbleibend hohe Qualität zu produzieren. Echtzeiterkennung bedeutet in diesem Zusammenhang, dass die Erkennung von fehlerhaften Elektroden-Separator-Verbunden innerhalb nur weniger Millisekunden erfolgt und durch die Ausgabe des entsprechenden Auswertungsergebnisses sofort Maßnahmen zur Fehlerbeseitigung eingeleitet werden können. Diese Maßnahmen können beispielsweise darin bestehen, dass die fehlerhafte Elektroden-Separator-Verbunde als fehlerhaft gekennzeichnet und aus dem Herstellungsprozess ausgeschleust werden. Eine andere Maßnahme kann darin bestehen, dass die bei dem gewählten Verbindungsprozess der Funktionsschichten gewählten Parameter in Schritt b) verändert werden. Wird bei dem Verbindungsprozess beispielsweise die Lamination eingesetzt, können als Parameter der Druck und/oder die Temperatur angepasst werden.

Insbesondere wird zur Weiterbildung vorgeschlagen, dass im Schritt a) wenigstens eine Elektrode mit einer Aktivmaterialschicht und wenigstens eine Separatorschicht zusammengefügt werden. Hierbei besteht die Elektrode beispielsweise aus einem elektrisch leitenden Substrat und einer darauf aufgebrachten Schicht von Aktivmaterial. Bei einer vorteilhaften Weiterbildung der Erfindung kann zudem vorgesehen sein, dass die Elektrode zuerst einzeln oder gleichzeitig während der Herstellung des Elektroden-Separator-Verbunds gefertigt wird, indem wenigstens eine Substratschicht und eine Aktivmaterialschicht als Funktionsschichten miteinander verbunden werden.

Eine andere Weiterbildung kann beispielsweise vorsehen, dass eine Anode und eine Kathode und zwei Separatoren gleichzeitig in einem Herstellungsschritt miteinander verbunden werden. Dies würde einem vierschichtigen Aufbau des Elektroden-Separator-Verbunds entsprechen.

Grundsätzlich ist es möglich den Elektroden-Separator-Verbund in unterschiedlichsten Konfigurationen herzustellen. So kann der Elektroden-Separator-Verbund beispielsweise zweischichtig, dreischichtig oder vielschichtig aufgebaut sein. Zudem kann auch ein Stromableiter als sogenanntes Ableiterfähnchen direkt als Teil des Schichtaufbaus der Funktionsschichten mit einbezogen bzw. mit einlaminiert sein.

Elektrodenableiter sind dazu vorgesehen aus dem Zellstapel herauszuragen, um auf diese Weise den erzeugten Strom an einen außerhalb des Zellstapels befindlichen elektrischen Verbraucher abzugeben. Innerhalb sind Elektroden dazu vorgesehen, einen erzeugten Strom direkt oder über Stromableiter an die jeweils zugehörige und polungsgleiche Elektrodenableiter abzuleiten. Als Substratfolie können beispielsweise anodenseitig Kupferfolien eingesetzt werden, die in Kontakt mit einer Graphitbeschichtung stehen, welche ein niedriges Elektrodenpotenzial aufweist. Kathodenseitig können die Substratfolien beispielsweise als Aluminiumkollektoren ausgebildet sein, auf denen ein Mischoxid aufgebracht ist. Dabei können die Graphitanode als erstes Aktivmaterial und das Mischoxid als zweites Aktivmaterial verwendet werden. Ordnet man nun innerhalb einer Batteriezelle eine Vielzahl von Elektrodensheets an, so kann die Batteriezelle eine positiven und eine negativen Elektrodenableiter aufweisen und die übrigen Elektroden-Separator-Verbunde können über Ableiterfähnchen mit dem jeweils entsprechenden positiven oder negativen Elektrodenableiter verbunden sein. Bei einem mehrschichtigen Aufbau des Elektroden-Separator-Verbunds sind entsprechende Separatorschichten vorzusehen, die einen elektrischen Kontakt zwischen den Elektroden-Separator-Verbunden unterschiedlicher Polung zuverlässig verhindern.

Insbesondere ist es vorteilhaft, wenn die Funktionsschichten mittels eines Laminier-, Klebe- oder Schweißverfahrens (großflächig) miteinander verbunden werden. Diese Verfahren lassen sich in der industriellen Großserienfertigung äußerst kostengünstig und prozesssicher umsetzen. Insbesondere das Laminierverfahren kann über die Parameter Zeit, Druck und Temperatur besonders genau auf die jeweiligen Anforderungen abgestimmt werden.

Ganz besonders vorteilhaft ist es, wenn die Schritte a) bis e) nacheinander in einem kontinuierlichen Durchlaufverfahren ausgeführt werden. Dadurch kann man die Vorteile der hohen Fertigungsgeschwindigkeit einer Durchlauffertigung mit kontinuierlicher Zuführung der Funktionsschichten und einer fortlaufenden Qualitätsüberwachung kombinieren. Kontinuierlich im Sinne der Erfindung umfasst sowohl kontinuierliche Abläufe der Zuführung mit einer konstanten Zufuhrgeschwindigkeit der Funktionsschichten, als auch solche kontinuierlichen Abläufe, bei denen die Funktionsschichten bzw. Abschnitte von Funktionsschichten mit einer konstanten Taktrate zugeführt werden. Entsprechendes gilt für die Abfuhrgeschwindigkeit bzw. Taktrate der Abfuhr der überprüften fertigen Elektroden-Separator-Verbunde. Insbesondere können auch bereits vorgefertigte Elektroden-Separator-Verbunde der Überprüfung zugeführt werden, wobei dann der Schritt des Verbindens der Funktionsschichten entfallen kann.

Die Erfassungsvorrichtung kann unmittelbar nach dem Verbinden der Funktionsschichten zumindest ein Teil der Oberfläche des gefertigten Elektroden-Separator-Verbunds erfassen und anschließend ein Messergebnis erzeugen, welches maschinell auswertbar ist und während dieser Auswertung ein Auswertungsergebnis erzeugt. Dieses Auswertungsergebnis wird dann fortlaufend ausgegeben, sodass ein menschlicher Bediener oder eine angeschlossene automatisierte Maschinensteuerung entsprechend dem Auswertungsergebnis eine Aktion zur Fehlerbeseitigung ausführen kann.

Sobald beispielsweise das Auswertungsergebnis angibt, dass die Verbindung der Funktionsschichten miteinander nicht mehr den vorgegebenen Anforderungen genügt, kann die Produktion gestoppt und die fehlerbehafteten Abschnitte des Elektroden-Separator-Verbunds entfernt werden. Wie bereits zuvor erwähnt kann dies durch einen Bediener manuell erfolgen oder automatisiert von einer Maschinensteuerung ausgeführt werden. Je nach gewählter Erfassungsvorrichtung kann die Oberfläche des erzeugten Elektroden-Separator-Verbunds vollständig, d. h. beidseitig oder nur teilweise, d. h. beispielsweise einseitig erfasst werden. Neben der einseitigen Erfassung der Oberfläche des Elektroden-Separator-Verbunds ist es möglich, dass nur bestimmte Teilbereiche des Elektroden-Separator-Verbunds von der Erfassungseinrichtung erfasst werden. So kann es bei bestimmten Anwendungsfällen bereits ausreichend sein, Teilbereiche des Elektroden-Separator-Verbunds bei der Erfassung zu überwachen, anstelle der gesamten Fläche bzw. der gesamten Breite des Elektroden-Separator-Verbunds.

Erfindungsgemäss wird die Erfassung der Oberfläche mittels eines optischen Kamerasystems durchgeführt. Mit einem Kamerasystem ist es in besonders einfacher Weise möglich eine Seite des gefertigten Elektroden-Separator-Verbunds zu überwachen, wenn das Kamerasystem stationär montiert ist und der Elektroden-Separator-Verbund in einem kontinuierlichen Prozess durch den Erfassungsbereich des Kamerasystems hindurchbewegt wird. Nicht erfindungsgemäss kann alternativ zu dem bildgebenden Verfahren mittels eines Kamerasystems die Erfassung mit anderen Systemen durchgeführt werden, wie beispielsweise Systeme, die mit sichtbarem oder unsichtbarem Licht, Radar, Laser oder Ultraschall arbeiten, um die erzeugte Verbindung der Funktionsschichten auf ihre Qualität hin zu überprüfen.

Erfindungsgemäß wird die Auswertung des Messergebnisses mittels eines elektronischen Datenverarbeitungssystems durchgeführt, welches zur Grauwertermittlung und/oder Erstellung eines Grauwert-Histogramms konfiguriert ist. Ein solches Datenverarbeitungssystems kann beispielsweise das von dem Kamerasystem erzeugte Messergebnis auswerten. Im Falle eines Kamerasystems handelt es sich bei dem Messergebnis um einzelne Bilder oder eine sich fortlaufend ändernde Abfolge von Bildern, die zur Auswertung an das Datenverarbeitungssystems weitergeleitet werden. Innerhalb des Datenverarbeitungssystems erfolgt die Auswertung entsprechend vorgegebener Regeln.

Insbesondere ist vorgesehen, dass bei der Auswertung des Messergebnisses ein zugehöriger Kennwert und insbesondere ein Grauwert, ermittelt wird. Dieser IST-Kennwert wird danach im Rahmen der Auswertung des Messergebnisses mit einem vorgegebenen SOLL-Kennwert verglichen, wobei durch einen Vergleich von IST-Kennwert und SOLL-Kennwert das Auswertungsergebnis bestimmt wird. Hierzu kann beispielsweise definiert werden, dass ein IST-Kennwert der größer oder gleich einem vorgegebenen SOLL-Kennwert ist, akzeptiert wird, während ein IST-Kennwert der kleiner als der vorgegebene SOLL-Kennwert ist, nicht akzeptiert wird. Wird der IST-Kennwert nicht akzeptiert, entspricht der betrachtete Abschnitt des Elektroden-Separator-Verbunds somit nicht den gestellten Qualitätsanforderungen.

Erfindungsgemäss wird das von dem Kamerasystem bereitgestellte Messergebnis einer Auswertung der Grauwerte unterzogen. Es ist nämlich zu beobachten, dass eine Korrelation zwischen dem Grauwert der Oberfläche des Elektroden-Separator-Verbunds und der Qualität der Verbindung zwischen den Funktionsschichten besteht. Grundsätzlich kann diese Korrelation so geschrieben werden, dass je besser die Haftverbindung zwischen den Funktionsschichten ist, desto höher ist der Grauwert des von dem Kamerasystem erzeugten Messergebnisses. Je höher wiederum der Grauwert ist, desto dunkler sind die Graustufen auf dem entsprechend zugehörigen Messergebnis bzw. Bild des Erfassungssystems. Es gibt jedoch ein Maximum ab der kein Anstieg der Haftfestigkeit mehr zu verzeichnen ist. Ab diesen Punkt kommt es sogar zu einem Abfall der Haftfestigkeit. Die Grauwertbildung der aufgenommenen Bilder wird mit einem Bildverarbeitungsprogramm durchgeführt. Hierbei wird ein Grauwerthistogramm erstellt, welches angibt, wie viele Pixel eines Bildes einen bestimmten Grauwert besitzen. Aus diesem Werten wird dann ein Mittelwert gebildet, der zum Vergleich der Proben genutzt wird. Aus dem aufgenommenen Bild des Kamerasystems, wird mit Hilfe der Funktion ein Bereich ausgewählt, der in die Grauwertbildung integriert werden soll. Dadurch wird der Grauwert nicht nur lokal bestimmt, sondern ein großflächiges Flächenintegral ermittelt, aus welchem dann der Mittelwert gebildet wird. Hierbei ist anzumerken, dass die ermittelten Grauwerte nur relative Grauwerte darstellen. Zur Ermittlung der exakten Grauwerte, kann eine vorherige Kalibrierung des verwendeten Bildverarbeitungssystems mit einem Graukeil durchgeführt werden.

Um möglichst gleichbleibende Messergebnisse zu erhalten ist es vorteilhaft, wenn Schritt c) unter gleichbleibenden und definierten Lichtverhältnissen ausgeführt wird. Dies kann beispielsweise dadurch erreicht werden, dass der zu erfassende Bereich des Elektroden-Separator-Verbunds mit einer künstlichen Lichtquelle ausgeleuchtet wird und, beispielsweise durch eine Abschirmung, gegen den Einfluss von äußeren Lichtquellen, wie etwa Tageslicht, abgeschirmt ist. Idealerweise ist dazu das Kamerasystem zusammen mit einer Beleuchtungseinheit innerhalb eines Gehäuses angeordnet. Auf diese Weise kann die Zuverlässigkeit des Auswertungsergebnisses deutlich gesteigert werden.

Die Aufgabenstellung wird ferner erfindungsgemäss durch eine Vorrichtung zur Überprüfung eines mehrschichtigen Elektroden-Separator-Verbunds für eine Batteriezelle gemäss dem unabhängigen Anspruch 8 gelöst.

Die Vorrichtung ist eingerichtet, das erfindungsgemässe Verfahren durchzuführen.

Es wird eine Vorrichtung mit mindestens einer Erfassungsvorrichtung und Mitteln vorgeschlagen, wobei diese geeignet sind, die hier erläuterten Schritte a) bis d) auszuführen. Weiter wird ein Computerprogramm[produkt] vorgeschlagen, umfassend Befehle, die bewirken, dass diese Vorrichtung die Schritte a) bis d) ausführt. Ebenso wird ein computerlesbares Medium vorgeschlagen, auf dem dieses Computerprogramm[produkt] gespeichert ist.

Mittels der derart gestalteten Vorrichtung kann die Überprüfung des gefertigten Elektroden-Separator-Verbunds kontinuierlich und mit hohen Geschwindigkeiten durchgeführt werden, wobei eine vollständige Prozessüberwachung in Echtzeit, eine sogenannte in-line-Überprüfung realisiert ist.

Hierdurch ist es möglich die Produktivität signifikant zu erhöhen, da Unterbrechungen zur Qualitätssicherung und zur Durchführung von Messungen nicht mehr erforderlich sind. Gleichzeitig kann die Ausschussrate deutlich reduziert werden, da fehlerhafte Verbindungen der Funktionsschichten unmittelbar und kurzzeitig nach der erfolgten Fertigung erkannt werden. Treten Fehler an den Verbindungen in dem Elektroden-Separator-Verbund auf, werden nur äußerst kleine Längen von fehlerhaften Elektroden-Separator-Verbunden gefertigt, bevor ein Benutzer oder eine Maschinensteuerung nach Ausgabe des Auswertungsergebnisses das System anhalten können bzw. Maßnahmen zur Fehlerbeseitigung ergreifen.

Weiterhin schlägt die vorliegende Offenbarung eine Batteriezelle vor, insbesondere eine Lithium-Ionen-Zelle, die wenigstens zwei Elektroden mit jeweils wenigstens einem Elektroden-Separator-Verbund aufweisen. Die Elektroden-Separator-Verbunde der unterschiedlichen Elektroden weisen einen wenigstens zweischichtigen Aufbau auf, sind gemäß dem beanspruchten Verfahren hergestellt und durch jeweils wenigstens eine Separatorschicht voneinander getrennt. Die so gefertigte und beschaffene Batteriezelle ist besonders kostengünstig zu fertigen und weist hervorragende elektrische Eigenschaften auf.

Vorsorglich sei angemerkt, dass die hier verwendeten Zahlwörter ("erste", "zweite", ...) vorrangig (nur) zur Unterscheidung von mehreren gleichartigen Gegenständen, Größen oder Prozessen dienen, also insbesondere keine Abhängigkeit und/oder Reihenfolge dieser Gegenstände, Größen oder Prozesse zueinander zwingend vorgeben. Sollte eine Abhängigkeit und/oder Reihenfolge erforderlich sein, ist dies hier explizit angegeben oder es ergibt sich offensichtlich für den Fachmann beim Studium der konkret beschriebenen Ausgestaltung.

Die Erfindung sowie das technische Umfeld werden nachfolgend anhand der beiliegenden Figuren näher erläutert. Es ist darauf hinzuweisen, dass die Erfindung durch die angeführten Ausführungsbeispiele nicht beschränkt werden soll. Insbesondere ist es, soweit nicht explizit anders dargestellt, auch möglich, Teilaspekte der in den Figuren erläuterten Sachverhalte zu extrahieren und mit anderen Bestandteilen und Erkenntnissen aus der vorliegenden Beschreibung zu kombinieren. Insbesondere ist darauf hinzuweisen, dass die Figuren und insbesondere die dargestellten Größenverhältnisse nur schematisch sind. Es zeigen:
Fig. 1: eine schematische Darstellung einer Vorrichtung zur Überprüfung eines Elektroden-Separator-Verbunds;
Fig. 2: eine Ausführungsform des vorgeschlagenen Verfahrens vom Laminieren bis zur Grauwertermittlung;
Fig. 3: ein Diagramm mit Korrelation von Grauwert und Haftfestigkeit; und
Fig. 4: ein Kraftfahrzeug mit Batteriezellen gemäß der vorliegenden Offenbarung

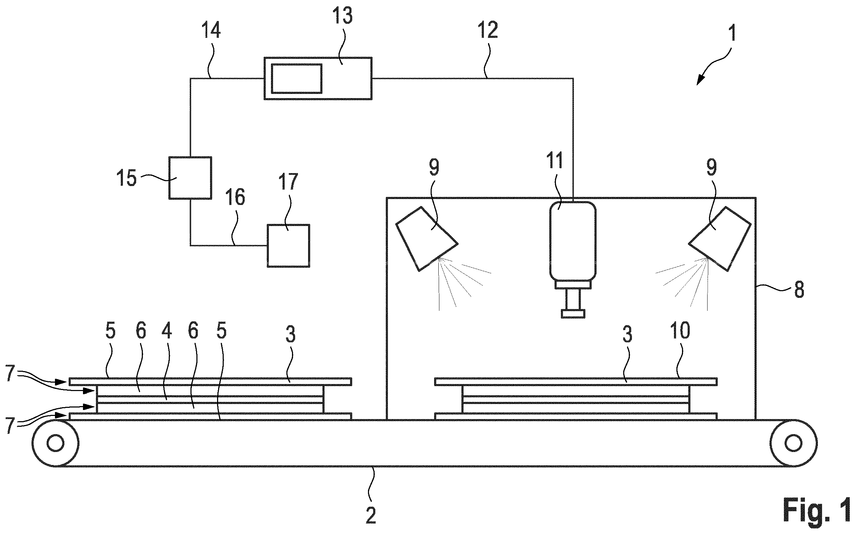

In Figur 1 ist eine Vorrichtung 1 zur Durchführung des hier erläuterten Verfahrens in einer schematischen Seitenansicht dargestellt.

Mittels einer Zuführvorrichtung 2 wird ein bereits laminierter Elektroden-Separator-Verbund 3 von links kommend zugeführt.

Der Elektroden-Separator-Verbund 3 weist bei der dargestellten Ausführungsform eine Elektrode 4 sowie zwei Separatorschichten 5 auf. Zwischen der Elektrode 4 und den Separatorschichten 5 befinden sich noch Aktivmaterialschichten 6. In dem dargestellten Zustand sind die Funktionsschichten 7 bestehend aus der Elektrode 4 mit den Aktivmaterialschichten 6 und den beiden Separatorschichten 5 bereits durch Laminieren miteinander verbunden. Die Lamination fügt dabei die Elektrode 4 und die darauf befindlichen Aktivmaterialien 6 mit dem Separator 5 zusammen. Die Verfahrensschritte a) und b) des Verfahrens sind damit bereits ausgeführt.

Im nächsten Schritt erfolgt das Erfassen wenigstens eines Teils der Oberfläche des laminierten Elektroden-Separator-Verbunds 3. Dazu wird der Elektroden-Separator-Verbund 3 in ein Gehäuse 8 verfahren. Das Gehäuse 8 schirmt den darin befindlichen Elektroden-Separator-Verbund3 gegen äußere Lichteinflüsse ab. Um gleichbleibende Lichtverhältnisse innerhalb des Gehäuses 8 zu erreichen, ist darin ein eigenes Beleuchtungssystem 9 vorgesehen, welches den Elektroden-Separator-Verbund 3 optimal und in stets gleichbleibender Weise ausleuchtet.

Bei der dargestellten Ausführungsform wird eine Oberfläche 10 des Elektroden-Separator-Verbunds 3 mittels eines Kamerasystems 11 erfasst. D. h. es wird nur ein Teilbereich der Oberfläche 10 des Elektroden-Separator-Verbunds 3 erfasst, nämlich die Oberseite, während die Unterseite nicht erfasst wird. Durch eine Öffnung (Fenster) im Fördersystem oder einem umgekehrten Vakuumband, könnte der Elektroden-Separator-Verbund alternativ oder zusätzlich auch auf der Unterseite untersucht und überprüft werden. Das von dem Kamerasystem 11 erfasste Messergebnis wird in Form eines Bildes über eine Signalleitung 12 an eine elektronische Datenverarbeitungseinrichtung 13 weitergeleitet.

Alternativ kann die Überprüfung auch durch fortlaufende Übertragung von Bildern und Messwerten auch in schneller zeitlicher Abfolge erfolgen und in Form einer Messwert- bzw. Videosequenz als Messergebnis bereitgestellt werden.

In der Datenverarbeitungseinrichtung 13 erfolgt dann eine Bildverarbeitung in Form einer Grauwertermittlung. Bei der Ermittlung des Grauwertes, wird für das von dem Kamerasystem 11 bereitgestellten Bild ein Grauwert ermittelt. Dieser Grauwert kann für das gesamte Bild oder für Teilbereiche des Bildes ermittelt werden. Nachdem wenigstens ein Grauwert ermittelt ist, wird dieser ermittelte IST-Grauwert mit einem vordefinierten Soll-Grauwert verglichen. In einem bestimmten Intervall der Grauwerte gilt dabei, je besser die Verbindung zwischen den Funktionsschichten 7 ist, desto höher ist der ermittelte IST-Grauwert, welcher von der Datenverarbeitungseinrichtung 13 für das erzeugte Bild bestimmt wird. Hierbei wird die Tatsache genutzt, dass je besser die Haftverbindung zwischen den Funktionsschichten 7 ist, desto dunkler wird das Bild und desto höher wird folglich der Grauwert, bis dieser schließlich ein Maximum erreicht.

Steigt der Grauwert darüber hinaus an, kann die Haftfestigkeit wieder absinken. Liegt also ein ermittelter IST-Grauwert exakt auf dem vorgegebenen SOLL-Grauwert oder darüber, so ist die Verbindung zwischen den Funktionsschichten 7 einwandfrei hergestellt. Werden dagegen bei den zu überwachenden Elektroden-Separator-Verbunden Grauwerte ermittelt, die unterhalb des Soll-Grauwertes liegen, so handelt es sich um nicht korrekte Verbindungen zwischen den Funktionsschichten 7 und die entsprechende Länge des Elektroden-Separator-Verbunds 3 ist fehlerhaft und als Ausschuss zu entsorgen. Das Ergebnis dieses Vergleichs zwischen dem ermittelten IST-Grauwert und dem vorgegebenen SOLL-Grauwert wird anschließend als Auswertungsergebnis ausgegeben. Dies kann beispielsweise mittels einer zweiten Signalleitung 14 zu einer SPS-Steuerung 15 und weiter über eine dritte Signalleitung 16 zu einem Server 17 erfolgen. Der Server 17 kann dabei wahlweise lokal betrieben werden oder als sogenannte Cloud-Lösung ausgebildet sein.

Mit der beschriebenen Vorrichtung können die Funktionsschichten bzw. Zellkomponenten, wie Anoden, Separatorschichten oder Kathoden fest miteinander verbunden werden, wobei diese sehr präzise zueinander positioniert und orientiert angeordnet werden können. Durch das Verbinden der Funktionsschichten bzw. Zellkomponenten entsteht ein sogenannter Elektroden-Separator-Verbund. Dieser kann insbesondere bei großformatigen Batteriezellen eingesetzt werden. Solche Batteriezellen werden künftig in hohen Stückzahlen benötigt, weshalb deren Fertigung mit hohen Prozessgeschwindigkeiten von besonderem Vorteil ist, um die Kosten zu senken und die geforderte Qualität zu erreichen.

Ferner können auch die Leistungswerte von Batteriezellen verbessert werden, die wie hier vorgeschlagen gefertigt werden. Dies verbessert dann im Weiteren auch die Eigenschaften der fertigen Batterien in welche Batteriezellen mit solchen Elektroden-Separator-Verbunden einfließen.

Die teilweise sehr sensiblen Materialien, welche bei der Herstellung von Batteriezellen verarbeitet werden müssen, können mit dem vorliegenden Verfahren und der vorliegenden Vorrichtung sicher und schnell verarbeitet werden und gleichzeitig wird die unerwünschte Fertigung von großen Mengen fehlerhafter Elektroden-Separator-Verbunde vermieden. Hierzu wird die bereitgestellte in-line-Überprüfung genutzt, um die Qualität der Verbindung zwischen den Funktionsschichten zu bestimmen. Im Gegensatz zu den bisher im Stand der Technik bekannten Verfahren muss der laufende Fertigungsprozess nicht mehr mittels Einzelproben analysiert werden, sondern es kann eine fortlaufende Analyse in Echtzeit erfolgen. Mögliche Defizite im Herstellungsverfahren werden somit unmittelbar und nicht erst nach Vollendung des Produktes entdeckt. Hierdurch lassen sich die Kosten für die Herstellung senken und die Effizienz des Produktionsprozesses wird deutlich verbessert. Es wird die Erkenntnis genutzt, dass Untersuchungen eine Korrelation zwischen der Oberflächenhelligkeit bzw. dem Grauwert der laminierten Elektroden-Separator-Verbunde und den im Fertigungsprozess verwendeten Laminationsparametern wie Druck und Temperatur erkannt haben. Mit einer Steigerung der Kompressionsrate steigt auch der Grauwert an. Proben von Elektroden-Separator-Verbunden, die kurz auch als Laminate bezeichnet werden können, die einer höheren Kompression ausgesetzt sind, erscheinen somit "dunkler", da beispielsweise die Oberfläche der Kathode stärker durch die Separatorschicht durchscheint. Gleiches gilt für die Änderung der Temperaturen. Auch hier erfolgt ein Absenken des Grauwertes durch Erhöhung der Temperatur während des Laminationsprozesses. Da die beiden Parameter Druck und Temperatur zusätzlich in Korrelation mit der Haftfestigkeit der laminierten Elektroden-Separator-Verbunds stehen, kann dadurch die Qualität dieser Haftfestigkeit ebenfalls bewertet werden. Über eine Grauwertermittlung des Elektroden-Separator-Verbundes ist es somit möglich direkt die Haftfestigkeit zu bestimmen. Auf diese Weise kann direkt nach dem Laminationsprozesses eine Aussage über die Qualität des Zwischenproduktes in Gestalt des jeweils überprüften Elektroden-Separator-Verbunds getroffen werden, ohne eine zerstörende Prüfprozedur durchführen zu müssen. Zusätzlich kann die Vorrichtung bzw. die Datenverarbeitungseinrichtung mit einer optischen Erkennung von Fehlstellen ausgestaltet sein, um die Lamination in-line, d.h. im laufenden Fertigungsprozess zu kontrollieren. Auf diese Weise können sowohl großflächig unlaminierte Fehlstellen als auch lokale Fremdpartikel erkannt werden, wodurch eine vollständige Qualitätskontrolle des Elektroden-Separator-Verbunds möglich ist.

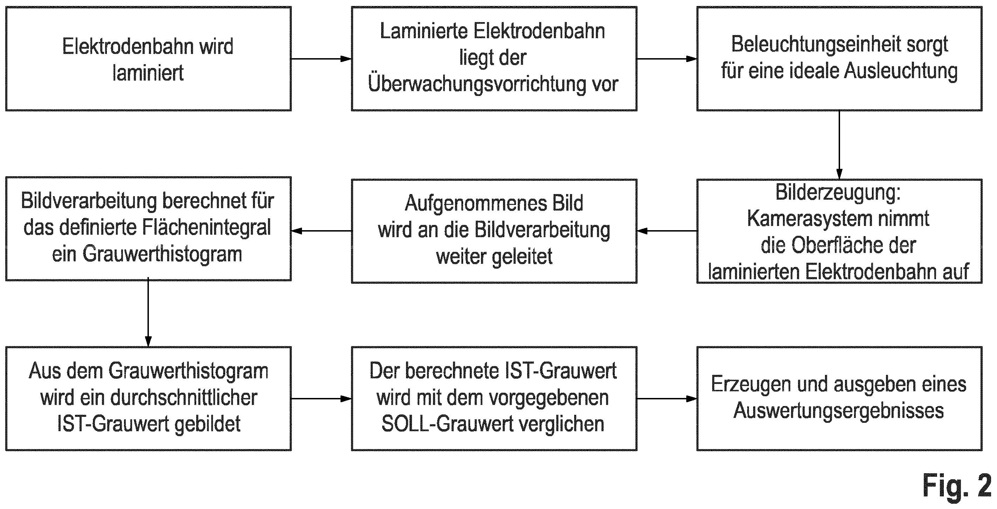

In Figur 2 sind die Schritte des Verfahrens ab dem Laminieren der Elektrode dargestellt. Danach wird die laminierte Elektrode zunächst dem Messsystem zugeführt. Dort erfolgt eine optimale Ausleuchtung durch ein Beleuchtungssystem, um anschließend im nächsten Schritt mit einem Kamerasystem ein Messergebnis zu erzeugen. Danach wird das Messergebnis in Form eines Bildes einer Bildverarbeitung in einer Datenverarbeitungseinrichtung zugeführt, wo eine Ermittlung des IST-Grauwerts erfolgt. Das Bild kann dabei ein Farbbild oder ein Graustufenbild sein. Dies geschieht mittels eines Graustufenhistogramms für das definiertes Flächenintegral. In einem weiteren Schritt wird aus dem Graustufenhistogramm ein durchschnittlicher IST-Grauwert gebildet, der danach in einem nächsten Schritt mit einem vorgegebenen SOLL-Grauwert verglichen wird. Im letzten Schritt wird aus dem Vergleich des IST-Grauwerts mit dem SOLL-Grauwert ein Auswertungsergebnis erzeugt und ausgegeben. Die Ausgabe kann dazu in visueller, optischer, haptischer, akustischer oder anderer Form erfolgen, die dazu geeignet ist, einem menschlichen Anwender das Auswertungsergebnis zu vermitteln. Bei einer besonders einfachen Ausführungsform kann das Auswertungsergebnis als binärer Wert, wie etwa, gut/schlecht, ja/nein ausgegeben werden und so den Anwender darüber informieren, ob der Elektroden-Separator-Verbund den Anforderungen entspricht oder nicht. Bei einer anderen Form der Ausgabe des Auswertungsergebnisses kann dies in Form von Signalen erfolgen, die zur Weiterverarbeitung in einer Datenverarbeitungseinrichtung bestimmt sind.

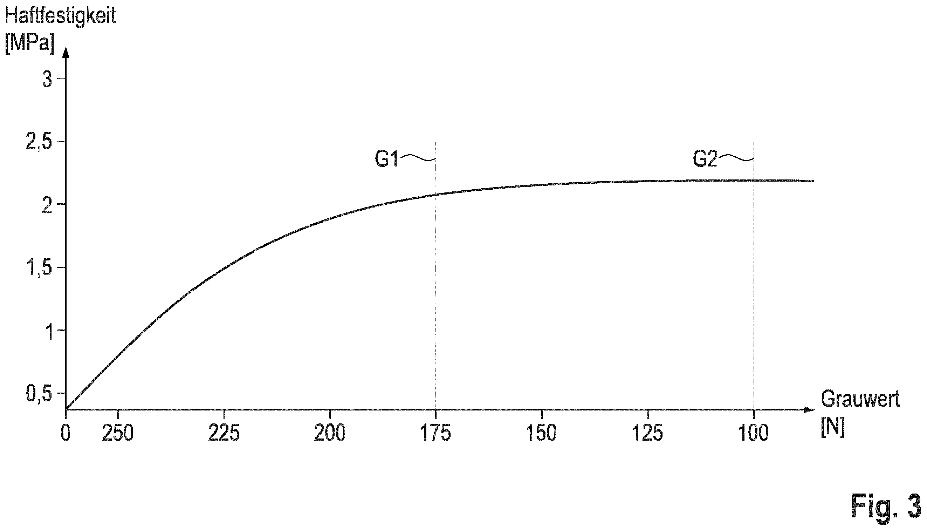

In Figur 3 ist an einem konkreten Beispiel der Zusammenhang zwischen dem Grauwert und der Haftfestigkeit zwischen den Funktionsschichten 7 qualitativ dargestellt. Gut erkennbar ist in dieser Figur, dass ab einem Grauwert G1 von ca. 175 [N] die Haftfestigkeit bereits einen sehr hohen Wert erreicht, der ca. 90% eines Maximalswertes erreicht, welchen die Haftfestigkeit am Grauwert G2 erreicht. Somit kann beispielsweise der Wert 175 als Soll-Wert dienen, ab dem eine ausreichend gute Verbindung zwischen den Funktionsschichten 7 besteht. Folglich können alle Elektroden-Separator-Verbunde 3 mit einem Grauwert, der größer als dieser Soll-Wert G1 von 175 [N] und kleiner als der Soll-Wert G2 von 100 [N], ist als korrekt gefertigte Teile gekennzeichnet und weiterverarbeitet werden. Teile, die jedoch einen Grauwert aufweisen die unter diesem Soll-Wert G1 oder über dem Soll-Wert G2 liegen, können unmittelbar als defekte Teile gekennzeichnet und dem Produktionsprozess entnommen werden. Alternativ können ausgehend vom Soll-Wert G2, der dem Maximum der Haftfestigkeit entspricht, auch ein Intervall ausgewählt werden in dem die Haftfestigkeit wenigstens 80%, vorzugsweise wenigstens 90% der maximalen Haftfestigkeit beträgt. Hierzu könnte das Intervall beispielsweise so gewählt werden, dass es von dem linksseitig von G2 liegenden unteren Soll-Wert G1 zu einem rechtsseitig von G2 liegenden oberen Soll-Wert G3 (in der Figur nicht dargestellt) reicht. Liegt nun der ermittelte Ist-Wert des Grauwerts in diesem Bereich, ist sichergestellt, dass die Haftfestigkeit wenigstens 80% und vorzugsweise wenigstens 90% der maximalen Haftfestigkeit beträgt.

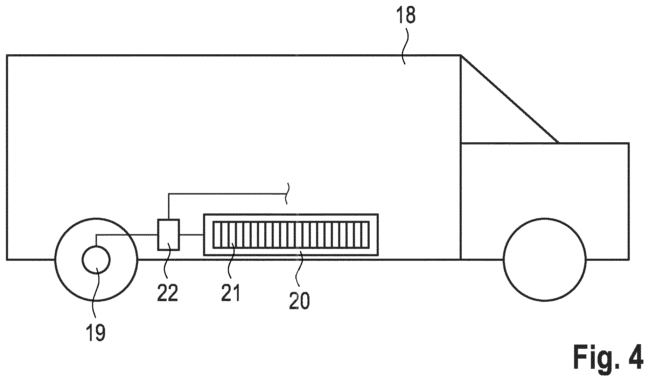

In Figur 4 ist schließlich noch ein Kraftfahrzeug 18 dargestellt, welches einen elektrischen Antrieb aufweist. Der elektrische Antrieb besteht aus einem Elektromotor 19 der mittels elektrischer Energie betrieben wird, die von einer Batterie 20 bereitgestellt wird. Die Batterie 20 wiederum weist eine Vielzahl von Batteriezellen 21 auf. Gesteuert wird die in Energieabgabe der Batterie 20 an den Elektromotor 19 mittels einer Steuerungseinrichtung 22. Die in der Batterie 20 angeordneten Batteriezellen 21 sind dabei mit Elektroden-Separator-Verbunden 3 gemäß der vorliegenden Erfindung ausgestattet und weisen damit die Vorteile auf, dass die Funktionsschichten 7 besonders zuverlässig verbunden sind, die Batterie 20 eine verbesserte Leistungsfähigkeit aufweist und die Herstellkosten reduziert sind.

### Bezugszeichenliste

- 1: Vorrichtung
- 2: Zuführvorrichtung
- 3: Elektroden-Separator-Verbund
- 4: Elektrode
- 5: Separatorschichten
- 6: Aktivmaterialschichten
- 7: Funktionsschichten
- 8: Gehäuse
- 9: Beleuchtungssystem
- 10: Oberfläche
- 11: Kamerasystem
- 12: Signalleitung
- 13: elektronische Datenverarbeitungseinrichtung
- 14: zweite Signalleitung
- 15: SPS-Steuerung
- 16: Dritte Signalleitung
- 17: Server
- 18: Kraftfahrzeug
- 19: Elektromotor
- 20: Batterie
- 21: Batteriezelle
- 22: Steuerungseinrichtung

## Patentansprüche

1. Verfahren zur Überprüfung eines mehrschichtigen Elektroden-Separator-Verbunds (ESV) (3) für eine Batteriezelle (21), welches die folgenden Schritte aufweist:
a) Zusammenfügen von wenigstens zwei Funktionsschichten (7) unter Ausbildung einer Haftverbindung, wobei die Funktionsschichten (7) schicht- oder plattenförmige Kathoden, Anoden oder Separatoren sind;
b) Verbinden der Funktionsschichten (7) zu einem mehrlagigen Elektroden-Separator-Verbund (3);
c) Erfassen wenigstens eines Teils einer Oberfläche (10) des Elektroden-Separator-Verbunds (3) mittels einer als ein Kamerasystem ausgeführten Erfassungsvorrichtung (11) zur Erzeugung eines Messergebnisses;
d) Auswerten des erzeugten Messergebnisses und Erzeugung eines Auswertungsergebnisses;
e) Ausgabe des Auswertungsergebnisses;
**dadurch gekennzeichnet, dass** die Auswertung des Messergebnisses mittels eines elektronischen Datenverarbeitungssystems (13) durchgeführt wird, welches zur Grauwertermittlung konfiguriert ist.

2. Verfahren zur Überprüfung eines Elektroden-Separator-Verbunds (3) für eine Batteriezelle (21) nach dem vorhergehenden Anspruch, **dadurch gekennzeichnet, dass** im Schritt a) wenigstens eine Elektrode mit einer Aktivmaterialschicht und wenigstens eine Separatorschicht zusammengefügt werden.

3. Verfahren zur Überprüfung eines Elektroden-Separator-Verbunds (3) für eine Batteriezelle (21) nach dem vorhergehenden Anspruch, **dadurch gekennzeichnet, dass** die Funktionsschichten mittels eines Laminier-, Klebe- oder Schweißverfahrens miteinander verbunden werden.

4. Verfahren zur Überprüfung eines Elektroden-Separator-Verbunds (3) für eine Batteriezelle (21) nach dem vorhergehenden Anspruch, **dadurch gekennzeichnet, dass** die Schritte a) bis e) nacheinander in einem kontinuierlichen Durchlaufverfahren (in-line) ausgeführt werden.

5. Verfahren zur Überprüfung eines Elektroden-Separator-Verbunds (3) für eine Batteriezelle (21) nach dem vorhergehenden Anspruch, **dadurch gekennzeichnet, dass** die Erfassung der Oberfläche (10) mittels eines optischen Kamerasystems (11) durchgeführt wird.

6. Verfahren zur Überprüfung eines Elektroden-Separator-Verbunds (3) für eine Batteriezelle (21) nach dem vorhergehenden Ansprüche, **dadurch gekennzeichnet, dass** bei der Auswertung zu dem Messergebnis ein zugehöriger IST-Kennwert, insbesondere ein Grauwert, ermittelt wird, dieser Kennwert danach mit einem vorgegebenen SOLL-Kennwert verglichen wird und danach durch einen Vergleich von IST-Kennwert und SOLL-Kennwert das Auswertungsergebnis bestimmt wird.

7. Verfahren zur Überprüfung eines Elektroden-Separator-Verbunds (3) für eine Batteriezelle (21) nach dem vorhergehenden Anspruch, **dadurch gekennzeichnet, dass** der Schritt c) unter gleichbleibenden und definierten Lichtverhältnissen ausgeführt wird.

8. Vorrichtung zur Überprüfung eines mehrschichtigen Elektroden-Separator-Verbunds (3) für eine Batteriezelle (21), mit einer Zuführvorrichtung (2) für ein Elektrodensheet (3), wobei eine als ein Kamerasystem ausgeführte Erfassungsvorrichtung (11) zur Erfassung wenigstens eines Teils der Oberfläche (10) des Elektroden-Separator-Verbunds (3) vorgesehen ist, die ein der Erfassung entsprechendes Messergebnis erzeugt, sowie einer Auswertungsvorrichtung (13) zur Auswertung des Messergebnisses, die nach der erfolgten Auswertung ein Auswertungsergebnis ausgibt; **dadurch gekennzeichnet, dass** die Vorrichtung Mittel umfasst, die geeignet sind, die Schritte a) bis d) gemäß einem der vorhergehenden Ansprüche 1 bis 7 auszuführen.

## Claims

1. Method for inspecting a multilayer electrode-separator composite (ESV) (3) of a battery cell (21), the method has the following steps:
a) joining of at least two functional layers (7) by forming an adhesive connection , wherein the functional layers (7) are layered or plate-shaped cathodes, anodes, and separators;
b) connecting the functional layers (7) into a multilayer electrode-separator composite (3);
c) detecting at least a part of the surface (10) of the electrode-separator composite (3) by means of a detection device (11), being a camera system, for generating a measurement result;
d) evaluating the measurement result produced and generating an evaluation result;
e) outputting the evaluation result;
**characterized in that** the evaluation of the measurement result is performed by means of an electronic data processing system (13), wherein the electronic data processing system (13) is configured for determining grayscale values.

2. Method for inspecting an electrode-separator composite (3) of a battery cell (21) according to the previous claim, **characterized in that** in step a) at least one electrode with an active material layer and at least one separator-layer are joined.

3. Method for inspecting an electrode-separator composite (3) of a battery cell (21) according to the previous claim, **characterized in that** the functional layers are mutually connected by means of a lamination method, an adhesive method or a welding method.

4. Method for inspecting an electrode-separator composite (3) of a battery cell (21) according to the previous claim, **characterized in that** steps a) to e) are successively carried out in a continuous through-feeding method (in-line).

5. Method for inspecting an electrode-separator composite (3) of a battery cell (21) according to the previous claim, **characterized in that** detection of the surface (10) is performed by means of an optical camera system (11).

6. Method for inspecting an electrode-separator composite (3) of a battery cell (21) according to the previous claim, **characterized in that** during the evaluation the actual characteristic value related to the measurement result is determined, In particular a gray value, the characteristic value is then compared with a predetermined target characteristic value, and the evaluation result is then determined by comparing the actual characteristic value with the target characteristic value.

7. Method for inspecting an electrode-separator composite (3) of a battery cell (21) according to the preceding claim, **characterized in that** step c) is performed under constant and defined light conditions.

8. Device for inspecting a multilayer electrode-separator composite (3) of a battery cell (21), the device having a conveying device (2) for an electrode sheet (3), wherein a detection device (11), being a camera system, is foreseen for detecting of at least a part of the surface (10) of the electrode-separator composite (3), the detection device produces a measurement result corresponding to the detection, and the device has an evaluation device (13) for evaluating the measurement result which outputs an evaluation result after the evaluation is performed; **characterized in that** the device comprises means being adapted to perform steps a) to d) according to to any one of the preceding claims 1 to 7.

## Revendications

1. Procédé de contrôle d'un composé séparateur d'électrodes multicouche (ESV) (3) pour une cellule de batterie (21), qui comprend les étapes suivantes :
a) assemblage d'au moins deux couches fonctionnelles (7) avec formation d'une liaison adhésive, les couches fonctionnelles (7) étant des cathodes, des anodes ou des séparateurs en forme de couches ou de plaques ;
b) connexion des couches fonctionnelles (7) pour former un composé séparateur d'électrodes multicouche (3) ;
c) détection d'au moins une partie d'une surface (10) du composé séparateur d'électrodes (3) au moyen d'un dispositif de détection (11) conçu comme un système de caméra pour générer un résultat de mesure ;
d) évaluation du résultat de mesure généré et génération d'un résultat d'évaluation ;
e) sortie du résultat de l'évaluation ;
**caractérisé en ce que** l'évaluation du résultat de mesure est effectuée au moyen d'un système de traitement de données électronique (13) qui est configuré pour la détermination de la valeur grise.

2. Procédé de contrôle d'un composé séparateur d'électrodes (3) pour une cellule de batterie (21) selon la revendication précédente, **caractérisé en ce qu'**à l'étape a), au moins une électrode avec une couche de matériau actif et au moins une couche de séparateur sont assemblées.

3. Procédé de contrôle d'un composé séparateur d'électrodes (3) pour une cellule de batterie (21) selon la revendication précédente, **caractérisé en ce que** les couches fonctionnelles sont reliées les unes aux autres au moyen d'un procédé de stratification, de collage ou de soudage.

4. Procédé de contrôle d'un composé séparateur d'électrodes (3) pour une cellule de batterie (21) selon la revendication précédente, **caractérisé en ce que** les étapes a) à e) sont effectuées successivement dans un processus continu (en ligne).

5. Procédé de contrôle d'un composé séparateur d'électrodes (3) pour une cellule de batterie (21) selon la revendication précédente, **caractérisé en ce que** la détection de la surface (10) est effectuée au moyen d'un système de caméra optique (11).

6. Procédé de contrôle d'un composé séparateur d'électrodes (3) pour une cellule de batterie (21) selon la revendication précédente, **caractérisé en ce que** lors de l'évaluation du résultat de mesure, une valeur caractéristique réelle correspondante, en particulier une valeur de gris, est déterminée, cette valeur caractéristique est ensuite comparée à une valeur caractéristique cible prédéfinie et ensuite le résultat d'évaluation est déterminé par une comparaison de la valeur caractéristique réelle et de la valeur caractéristique cible.

7. Procédé de contrôle d'un composé séparateur d'électrodes (3) pour une cellule de batterie (21) selon la revendication précédente, **caractérisé en ce que** l'étape c) est réalisée dans des conditions d'éclairage constantes et définies.

8. Dispositif de contrôle d'un composé séparateur d'électrodes multicouche (3) pour une cellule de batterie (21), avec un dispositif d'alimentation (2) pour une feuille d'électrodes (3), un dispositif de détection (11) conçu comme un système de caméra étant prévu pour détecter au moins une partie de la surface (10) du composé séparateur d'électrodes (3), qui produit un résultat de mesure correspondant à la détection, ainsi qu'un dispositif d'évaluation (13) pour évaluer le résultat de la mesure, qui délivre un résultat d'évaluation après l'évaluation effectuée ; **caractérisé en ce que** le dispositif comprend des moyens qui sont adaptés pour exécuter les étapes a) à d) selon l'une quelconque des revendications précédentes 1 à 7.
